(19) Europäisches Patentamt
European Patent Office
Office européen des brevets

(11) **EP 4 575 543 A1**

(12) **EUROPEAN PATENT APPLICATION**

(43) Date of publication:
**25.06.2025  Bulletin 2025/26**

(21) Application number: **23383313.6**

(22) Date of filing: **18.12.2023**

(51) International Patent Classification (IPC):
**G01R 33/56** (2006.01)    **A61B 5/055** (2006.01)
**G06F 18/24** (2023.01)

(52) Cooperative Patent Classification (CPC):
**G01R 33/5608; A61B 5/055; G06F 18/23;
G06F 18/24;** G06F 2218/12

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Leiden University Medical Center
2333 ZA  Leiden (NL)**
• **Consejo Superior De Investigaciones Científicas
-
CSIC
28006 Madrid (ES)**
• **Universitat Politècnica de València
46022 Valencia (ES)**

(72) Inventors:
• **ALONSO OTAMENDI, Joseba
46022 Valencia (ES)**
• **GALVE CONDE, Fernando
46022 Valencia (ES)**
• **ALGARÍN GUISADO, José Miguel
46022 Madrid (ES)**
• **BENLLOCH BAVIERA, José María
46022 Valencia (ES)**
• **WEBB, Andrew
2333ZA Leiden (NL)**

(74) Representative: **TRBL Intellectual Property
Plaza de Castilla 3, 7°A
28046 Madrid (ES)**

(54) **METHOD OF CLASSIFICATION OF MAGNETIC RESONANCE SIGNALS, COMPUTER PROGRAM FOR CARRYING OUT SAID METHOD AND SYSTEM ADAPTED TO PERFORM SAID METHOD**

(57)  The present invention relates to a method of classification of magnetic resonance, MR, signals indicative of one or more portions of a subject or a sample of diagnostic or prognostic interest, realized by means of an NMR scanner (13) and a processing means. The method comprises acquiring, with the NMR scanner (13), a set of MR signal data (7, 7') for each one or more MR signals emitted from the subject or the sample (14). The one or more MR signal data sets (7, 7') comprise one-dimensional time-domain signal data and are processed by the processing means. The processing means compares the one or more MR signal data sets (7, 7') with reference data, and classifies the one or more MR signals in one or more diagnostic or prognostic interest groups. The reference data comprise one-dimensional time-domain signal data. The present invention also relates to a system and a computer program adapted for carrying out said method.

FIG. 1

EP 4 575 543 A1

**Description**

**FIELD OF THE INVENTION**

The present invention relates to the field of magnetic resonance (MR) diagnostic techniques. More specifically, the invention relates to a method for classifying MR signals indicative of portions of diagnostic or prognostic interest acquired from a subject or a sample, and to a system and a computer program adapted for carrying out said method.

**BACKGROUND ART**

Magnetic resonance (MR) is a non-invasive technique commonly used to obtain information from an object subjected to non-ionizing electromagnetic (EM) fields. Typically, in this technique, a nuclear MR (NMR) scanner is used to scan a subject or sample, by applying a main magnetic field and executing a sequence of EM fields and acquiring one or more MR signals emitted from said subject or said sample, which are preferably indicative of portions thereof of diagnostic or prognostic interest.

In the above context, common NMR scanners comprise a main magnet and a RF system, wherein the RF system comprises a number of RF transmitters and RF receivers. The main magnet applies the main magnetic field on the subject or the sample, polarising nuclear spins comprised in a portion of the subject or the sample. As a result, in that portion, a net magnetization of the polarised nuclear spins (that is, a vector sum of the angular momenta of nuclear spins) aligns with the main magnetic field. The RF transmitter is configured to excite the subject or the sample by executing at least one resonant RF field on the subject or the sample and, as a result, the net magnetization is rotated or tipped with respect to the direction of the main magnetic field. When the execution of a resonant RF field ceases, the net magnetization processes around the main magnetic field, at a frequency known as the Larmor frequency. The precession of the net magnetization leads to the emission of an MR signal in the form of RF radiation, which can be detected by the RF receiver. In general, the RF receiver comprises a resonant circuit to detect the MR signal through an alternating current signal (generally known as free induction decay (FID) signal). Typically, the resonant frequency of the resonant circuit is the aforementioned Larmor frequency. In addition, common NMR scanners are also configured to record the MR signal detected by the RF receiver, so that at least part of the emitted MR signal is acquired.

Common NMR scanners further comprise a magnetic gradient system, configured to execute one or more inhomogeneous magnetic field gradients on the subject or the sample, superimposed to the main magnetic field. As a consequence, the resonant frequency (i.e., the Larmor frequency) of the net magnetizations comprised in the subject or the sample depend on the position of said net magnetizations in the subject or the sample. That is to say, the execution of the one or more inhomogeneous magnetic fields, splits the subject or the sample in portions, where each portion comprises a net magnetization associated to a particular Larmor frequency. Only when the radiation frequency of an executed RF field is resonant with said particular Larmor frequency, or when at least said RF field has a radiation frequency bandwidth that includes said particular Larmor frequency, the executed RF field is resonant and rotates or tips the associated net magnetization.

MR imaging (MRI) methods are remarkably successful to obtain high-quality images of the internal spatial structure of a subject or a sample (typically, a human or animal body, or a part thereof). Said images are reconstructed from the MR signals acquired with NRM scanners. Said high-quality images enable the radiologists to easily and accurately identify the presence/absence of an actual or eventual pathology. In MRI methods, the acquired MR signal data are arranged as k-space data in a k-space, which are used to reconstruct images of the subject or the sample by means of mathematical tools typically involving Fourier transformations.

Despite their success, common MRI methods present several limitations. On the one hand, the acquired k-space data are constrained by the requirements associated to the reconstruction of a reliable image of the subject/sample. Said requirements typically imply that only little amounts of each emitted MR signals comprising information of the subject/sample are acquired for latter high-quality image reconstruction. Furthermore, large scan times are required to acquire the necessary amount of data and avoid image artifacts to reconstruct said high-quality images. In this context, said large scan times involve the execution of hundreds of EM sequence blocks comprising resonant RF pulses, and repetition times between consecutive EM sequence blocks of the order of 100 ms. Moreover, the quality of the images is directly dependent on the quantity and the quality of the acquired MR signal data arranged in a k-space, so that highly demanding hardware resources are generally required for the NMR scanners. As a result, NMR scanners are generally expensive.

On the other hand, nowadays the use of data-processing Artificial Intelligence (AI) algorithms has considerably increased in the field of MR methods. For instance, machine learning (ML) has been incorporated into MRI methods, such that the prior knowledge acquired from big datasets leads to a fast and accurate image reconstruction from limited measurements.

Furthermore, in recent years different AI-based methods and systems for classifying MR signals have been developed, wherein said methods are not based on an image reconstruction of a subject or a sample. These known methods are so-called "imageless methods".

An example of an imageless medical imaging method is disclosed in the international patent application WO 2019/051411 A1. This patent application discloses a ML-based method for analysing medical image data obtained from a subject with an imaging system. Therein, examples of imaging systems are computed tomography (CT) systems, magnetic resonance (MR) systems, ultrasound (US) systems, ultrasonic CT systems, positron emission tomography (PET) systems, single photon emission computed tomography (SPECT) systems, or x-ray imaging systems. The image data are received by a module comprised in a computing device. This module is based on a trained ML algorithm configured to directly operate on the image data, so that said data are analysed without requiring an image reconstruction from thereof. In particular, the image data received by the ML module are raw data, such as raw k-space data.

Another example of an imageless method for classifying MR signals and a system implementing said method is disclosed in Singhal et al., "On the Feasibility of Machine Learning Augmented Magnetic Resonance for Point-of-Care Identification of Disease", arXiv preprint arXiv:2301.11962 (2023). An example embodiment therein comprises a prostate-cancer-triaging system used in patients with a high prostate-specific antigen (PSA) score. Said system comprises an NMR scanner, and embedded processing means configured with an ML algorithm. The prostate-cancer-triaging system does not have to produce an image, but just a risk score. In this context, the ML algorithm disclosed in Singhal et al infers the presence/absence of a disease directly from the k-space data obtained by a NMR scanner, skipping the image reconstruction process. Instead, the ML algorithm identifies the subsets of the k-space data that have the largest predictive signal pertaining to the disease being inferred and, subsequently, applies a Deep Leaning (DL) classifier to infer the presence of the disease from said k-space data subsets. To perform the classification task, the ML algorithm is previously trained with sets of k-space data of MR signals obtained from a plurality of subjects.

A problem present in the prior-art imageless methods for classifying MR signals, such as those disclosed in WO 2019/051411 A1 and Singhal et al, is that the processed MR signal data follow from the application of main magnetic fields, the execution of EM sequences and the acquisition of MR signal data as typically realized in MRI methods. Consequently, known imageless methods, as well as systems implementing thereof, suffer from the same limitations of MRI methods. Thus, known imageless methods are constrained by the above high-demanding requirements of MRI methods associated to the reconstruction of a reliable image from the acquired k-space data. In particular, prior-art methods suffer from the use of little amounts of k-space data for each MR signals comprising information of the subject/sample and the need of large scan times. In the end, said constraining requirements compromise the accuracy of prior-art imageless methods for classifying MR signals.

In view of the above, there is a need in the field of MR techniques for providing alternative imageless methods and systems for classifying MR signals.

## SUMMARY OF THE INVENTION

The present invention overcomes the above limitations of the prior art, by providing an improved imageless method for classifying MR signals, and a system adapted to perform said method. The method of the invention is based on the processing, by a processing means, of MR signal data sets acquired with a NMR scanner, wherein said acquired MR signal data sets comprise data that are not suitable for image reconstruction. The acquisition of data that are not suitable for image reconstruction is typically discarded in traditional MRI methods and in known imageless MR methods. However, these data can, in fact, be used to effectively classify MR signals indicative of portions of a subject or sample of diagnostic or prognostic interest, to increase the amount of data acquired per MR signal emitted from the subject or the sample, and to reduce scan times, compared to the known techniques.

Hence, the present invention relates in a first aspect to a method of classification of magnetic resonance, MR, signals indicative of one or more portions of a subject or a sample of diagnostic or prognostic interest, comprising the realization, by means of an NMR scanner and a processing means, of the following steps:

a) applying a main magnetic field and executing a sequence of electromagnetic fields, on the subject or the sample arranged in the NMR scanner;

b) acquiring with the NMR scanner, during the application of the main magnetic field and the execution of the EM sequence in step a), at least part of each of the one or more MR signals emitted from the subject or the sample that result from the application of the main magnetic field and the execution of the EM sequence, so that a set of MR signal data is acquired for said each of the one or more emitted MR signals;

c) comparing, with the processing means, the one or more MR signal data sets of step b) with reference data, wherein said reference data are associated with one or more reference MR signals associated with one or more reference EM sequences for one or more reference subjects or samples, and wherein said reference data are segmented into a plurality of diagnostic or prognostic interest groups;

d) determining, with the processing means, one or more values of similarity between the diagnostic or prognostic interest groups and the one or more MR signal data sets;

e) classifying, with the processing means, the one or more MR signals emitted from the subject or the sample in one or more diagnostic or prognostic interest groups of the reference data from the values of similarity determined in step d).

Advantageously, in the present invention

- the one or more MR signal data sets comprise one-dimensional time-domain signal data; and
- the reference data associated with the one or more reference MR signals comprise one-dimensional time-domain signal data.

The one-dimensional (1D) time-domain signal data of the one or more data sets and of the reference data are respectively associated with the EM sequence executed in step b) and the one or more reference EM sequences. Herein the EM sequence executed in step b) and each of the one or more reference EM sequences comprise, at least, one resonant RF field. Hence, 1D time-domain signal data is understood as 1D time-dependent signal data associated with an MR signal, wherein said data are not rearranged into a 2D or 3D k-space that could produce, by Fourier transformation, an image of the internal spatial structure of the subject or the sample. Accordingly, the 1D time-domain signal data as defined in the present invention correspond to untransformed 1D time-dependent MR signals, and not the image raw data in the arranged domain used in the prior art (referred as raw k-space data, for instance, in the patent application WO 2019/051411 A1).

The acquisition of 1D time-domain signal data is discarded both in MRI techniques and known imageless methods for classifying MR signals (such as the methods disclosed in the patent application WO 2019/051411 A1, and in Singhal et al.). Furthermore, in known imageless MR methods, such as those based on AI algorithms proposed in WO 2019/051411 A1 and in Singhal et al., neither use reference data comprising 1D time-domain signal data for classifying MR signals. However, and even though 1D time-domain signal data are not suitable for reconstructing an image of the subject or the sample, the 1D time-domain signal data comprise information associated to said subject or said sample that is indeed relevant for the classification of the MR signals. Hence, the accuracy of the method according to present invention is enhanced with respect to known imageless methods for classifying MR signals.

In a second aspect, the invention relates to a computer program comprising instructions which, when the computer program is executed by a processing means, cause the processing means to carry out a method according to the first aspect of the invention.

Finally, in a third aspect the present invention relates to a system comprising an NMR scanner and a processing means communicatively coupled with the NMR scanner, wherein the NMR scanner and the processing means comprise hardware and/or software means adapted to perform a method according to the first aspect of the invention.

**BRIEF SUMMARY OF THE FIGURES**

Figure 1 shows a flowchart schematically illustrating a particular embodiment of a method for classifying MR signals according to the present invention.

Figure 2 shows an EM sequence according to a particular embodiment of the present invention.

Figure 3 shows an EM sequence according to another particular embodiment of the present invention.

Figure 4 shows a schematic representation of an NMR scanner according to a particular embodiment of the present invention.

Reference numbers used in the figures

| | | |
|---|---|---|
| (1) | Inputting a prescribed symptom input information | |
| (2) | Selecting EM sequences from the prescribed symptom input information | |
| (3) | Executing EM sequences and acquiring MR signal data sets | |
| (4) | Providing MR signal data sets to the processing means, and performing comparison and similarity determination steps | |

(continued)

| (5) | Classifying MR signals |
|---|---|
| (6) | EM sequence |
| (7, 7') | MR signal data sets |
| (8, 8') | EM sequence blocks of the EM sequence |
| (9, 9') | First resonant RF pulses of the EM sequence blocks |
| (10, 10') | Second resonant RF pulses of the EM sequences blocks |
| (11, 11') | Inhomogeneous magnetic field gradient pulses of the EM sequence blocks |
| (12) | Acquisition time window |
| (13) | NMR scanner |
| (14) | Sample |
| (15) | Main magnet |
| (16) | RF system |

## DETAILED DESCRIPTION OF THE INVENTION

As explained above, the present invention overcomes the above limitations of the prior art, by providing an improved imageless method for classifying MR signals, and a system adapted to perform said method. The method of the invention is based on the processing, by a processing means, of MR signal data sets acquired with an NMR scanner and comprising one-dimensional (1D) time-domain signal data. In said processing, the processing means compare the above MR signal data sets with reference data also comprising 1D time-domain signal data. Herein, 1D time-domain signal data is understood as 1D time-dependent signal data associated to an MR signal, wherein said data are not rearranged into a 2D or 3D k-space that could produce, by Fourier transformation, an image of the internal spatial structure of the subject or the sample.

Hence, the present invention relates, in a first aspect thereof, to a method of classification of magnetic resonance, MR, signals indicative of one or more portions of a subject or a sample of diagnostic or prognostic interest, comprising the realization, by means of a nuclear magnetic resonance, NMR, scanner and a processing means, of the following steps:

a) applying a main magnetic field and executing a sequence of electromagnetic, EM, fields, on the subject or the sample arranged in the NMR scanner;
b) acquiring with the NMR scanner, during the application of the main magnetic field and the execution of the EM sequence in step a), at least part of each of the one or more MR signals emitted from the subject or the sample that result from the application of the main magnetic field and the execution of the EM sequence, so that a set of MR signal data is acquired for said each of the one or more emitted MR signals. The one or more MR signal data sets comprise 1D time-domain signal data;
c) comparing, with the processing means, the one or more MR signal data sets of step b) with reference data, wherein said reference data are associated with one or more reference MR signals associated with one or more reference EM sequences for one or more reference subjects or samples, and wherein said reference data are segmented into a plurality of diagnostic or prognostic interest groups. In addition, the reference data associated with the one or more reference MR signals comprise 1D time-domain signal data;
d) determining, with the processing means, one or more values of similarity between the diagnostic or prognostic interest groups and the one or more MR signal data sets;
e) classifying, with the processing means, the one or more MR signals emitted from the subject or the sample in one or more diagnostic or prognostic interest groups of the reference data from the values of similarity determined in step d).

Herein, a sequences of EM fields (herein referred as "EM sequences") executed by the NMR scanner comprises at least one or more resonant radiofrequency (RF) fields. The term "resonant RF field" refers to a RF field whose radiation frequency is substantially equal to the resonant frequency (i.e., the Larmor frequency) of a net magnetization comprised in the subject or the sample. Herein, the expression "substantially" means that said radiation frequency is equal to said Larmor frequency, or equal to a radiation frequency bandwidth that includes said Lamor frequency.

The one or more MR signal data sets acquired by the NMR scanner are processed by a processing means, in order to classify the one or more MR signal emitted from the subject or the sample. As used herein, the term "processing means" refers to processing devices, apparatus, programs, circuits, components, systems, and/or subsystems, whether implemented in hardware, tangibly embodied software, or both, and whether or not it is programmable. Hence, according to what is described above, the processing means comprises hardware and/or software means adapted to perform the aforementioned steps c), d), and e).

In step c) the processing means compares the one or more acquired MR signal data sets with reference data associated with one or more reference MR signals. As used herein, the term "one or more reference MR signals" refers to one or more MR signals that exemplify and/or model the one or more MR signals acquired from one or more reference subjects or samples, and that result from the execution of one or more reference EM sequences. In addition, the reference data comprise medical information associated with the above one or more reference subjects or samples, so that said reference data is segmented into a plurality of diagnostic or prognostic interest groups. Further, in step d) the processing means determines one or more values of similarity between the diagnostic or prognostic interest groups and the one or more MR signal data sets, and in step e) it classifies the one or more MR signals emitted from the subject or the sample in one or more of the above diagnostic or prognostic interest groups. Hence, the present invention provides an imageless method for classifying MR signals emitted from a subject or a sample. As used herein, the term "imageless" means that said method of classification is not based on an image reconstruction of the subject or the sample.

Advantageously, compared to known imageless methods for classifying MR signals, in the method according to the present invention the one or more MR signal data sets comprise 1D time-domain signal data, and the reference data associated with the one or more reference MR signals also comprise 1D time-domain signal data. In the context of the present invention, the term "1D time-domain signal data" (as opposed to "raw k-space data") refers to 1D time-dependent signal data associated with an MR signal which are not rearranged into a 2D or 3D k-space that, through Fourier transformations, could produce images that reproduce the internal spatial structure of the subject or the sample.

In particular embodiments of the invention, the EM sequence executed in step a) comprises a single EM sequence block comprising a single resonant RF field, and a set of MR signal data is acquired from a single MR signal emitted from the subject or the sample in step b). In yet other particular embodiments of the invention, the above single EM sequence block further comprises one or more inhomogeneous magnetic field gradients. In the context of the present invention, the term "EM sequence block" refers to a part of the EM sequence comprising one or more resonant RF fields with same radiation frequency. Thus, in these particular embodiments of the invention, it is sufficient for the processing means to process the MR signal data set of a single emitted MR signal for classifying thereof. Advantageously, the scan times are considerably reduced with respect to MRI methods and known imageless MR methods, wherein typically hundreds of EM sequence blocks are executed on the subject or the sample, and the subsequent MR signal data sets are acquired. In particular embodiments of the invention, the above single resonant RF field executed in step a) is a continuous and/or time-modulated RF field executed during all step a), contrary to RF pulses commonly executed in known MR techniques.

In particular embodiments of the present invention, the EM sequence executed in step a) comprises a plurality of EM sequence blocks, wherein each EM sequence block comprises one or more resonant RF fields, so that the subject or the sample is repeatedly re-excited during step a). This allows to eventually avoid the inherent limitations (e.g., noise effects) of the short decaying time $T_2^*$ characterizing the MR signals (e.g., the decaying time $T_2^*$ is around 1 ms or less in hard tissues such as bones, teeth, etc.).

An inhomogeneous main magnetic field may be applied with an NMR scanner on a subject or a sample, so that the value of the resonant frequency (i.e., the Larmor frequency) of a net magnetization comprised in said subject or said sample, depends on the position of said net magnetization in the subject or the sample. In this scenario, a EM sequence comprising a plurality of EM sequence blocks may be executed with the above NMR scanner on the subject or the sample, so that said subject or said sample is repeatedly re-excited during said execution. Each EM sequence block comprises one or more resonant RF fields that only excite a portion of the subject or the sample. This means that the radiation frequency of said one or more resonant RF fields is resonant with the Larmor frequency of the net magnetization comprised in the above portion of the subject or the sample. On the contrary, the remaining portions of the of the subject or the sample are not excited by the one or more resonant RF fields comprised in the above EM sequence block, or they return to a relaxed state if resonant RF fields comprised in previously executed EM sequence blocks have already excited them. The situation describe in the present paragraph is herein referred as "multifrequency" or "high bandwidth" implementation. Multifrequency implementations can be realized in imageless methods for classifying MR signals, whether or not the acquired MR signal data sets and the reference data comprise 1D time-domain signal data.

Hence, according to the above, in particular embodiments of the invention (hereinafter referred as "multifrequency embodiments" or "high bandwidth embodiments"), aside from executing in step a) an EM sequence comprising a plurality of EM sequence blocks, where each EM sequence block comprises one or more resonant RF fields, so that the subject or the sample is repeatedly re-excited during step a):

- the main magnetic field applied in step a) is a non-uniform main magnetic field, so that a position-dependent Larmor frequency is imposed along the spatial extent of the subject or the sample along at least one direction in said subject or said sample, wherein the values of said position-dependent Larmor frequency along the spatial extent of the subject or the sample along said at least one direction are delimited by a bandwidth range ($\Delta w_L$) given by

$$(\gamma \Delta B_0)/(2\pi),$$

wherein $\Delta B_0$ is the difference ($\Delta B_0$) between the maximum amplitude ($B_{0,max}$) and the minimum amplitude ($B_{0,min}$) of the non-uniform main magnetic field along the spatial extent of the subject or the sample along the at least one direction in said subject or said sample, and $\gamma$ is the protonic gyromagnetic ratio;

- the one or more RF fields of each of the EM sequence blocks has a radiation frequency, wherein said radiation frequency is different for different EM sequence blocks and resonant with a single value of the position-dependent Larmor frequency along the spatial extent of the subject or the sample along the at least one direction in said subject or said sample.

In particular multifrequency embodiments, the difference ($\Delta B_0$) between the maximum amplitude ($B_{0,max}$) and the minimum amplitude ($B_{0,min}$) of the non-uniform main magnetic field along the spatial extent of the subject or the sample along the at least one direction in said subject or said sample, is equal or larger than 0.5 times the maximum amplitude ($B_{0,max}$) of the non-uniform main magnetic field.

In addition, in particular multifrequency embodiments, the ratio between the bandwidth range ($\Delta w_L$) delimiting the values of the position-dependent Larmor frequency along the spatial extent of the subject or the sample along the at least one direction in said subject or said sample, and the maximum value of the position-dependent Larmor frequency in said spatial extent of the subject or the sample along the at least one direction in said subject or said sample is

larger than 0.1, larger than 0.2, larger than 0.3, larger than 0.4, or larger than 0.5; and/or
equal to 0.1, 0.2, 0.3, 0.4, or 0.5.

In particular multifrequency embodiments, the bandwidth range ($\Delta w_L$) delimiting the values of the position-dependent Larmor frequency along the spatial extent of the subject or the sample along the at least one direction in said subject or said sample, is equal or larger than 100 kHz. In particular multifrequency embodiments, the bandwidth range delimiting the values of the position-dependent Larmor frequency along the spatial extent of the subject or the sample along the at least one direction in said subject or said sample

is between 100 kHz and 1000 kHz, between 150 kHz and 1000 kHz, between 200 kHz and 1000 kHz, between 250 kHz and 1000 kHz, between 300 kHz and 1000 kHz, between 350 kHz and 1000 kHz, between 400 kHz and 1000 kHz, between 450 kHz and 1000 kHz, between 500 kHz and 1000 kHz, between 550 kHz and 1000 kHz, between 600 kHz and 1000 kHz, between 650 kHz and 1000 kHz, between 700 kHz and 1000 kHz, between 750 kHz and 1000 kHz, between 800 kHz and 1000 kHz, between 850 kHz and 1000 kHz, between 900 kHz and 1000 kHz, or between 950 kHz and 1000 kHz; and/or
is equal to 100 kHz, 150 kHz, 200 kHz, 250 kHz, 300 kHz, 350 kHz, 400 kHz, 450 kHz, 500 kHz, 550 kHz, 600 kHz, 650 kHz, 700 kHz, 750 kHz, 800 kHz, 850 kHz, 900 kHz, 950 kHz, or 1000 kHz.

In particular embodiments of the invention, step b) comprises the record of the at least part of each of the one or more emitted MR signals detected by the NMR scanner. In particular embodiments of the invention, said NMR scanner detects the one or more emitted MR signals by means of a resonant circuit (for instance, a resonant circuit comprised in a RF receiver arranged in the NMR scanner). Typically, a resonant circuit is characterised by a quality factor or Q factor, given by the quotient of the resonant frequency of said resonant circuit and the frequency bandwidth of said resonant circuit. The Q factor measures the signal-to-noise-ratio (SNR) detected by the resonant circuit: the larger the Q factor, the larger the SNR of the detected emitted MR signal.

In the above context, in particular multifrequency embodiments,

- step b) comprises the record of the at least part of each of the one or more emitted MR signals detected by the NMR scanner by means of resonant circuit;
- the resonant circuit has a retunable resonant frequency, so that, for the detection of the emitted MR signal resulting from the execution of each EM sequence block, the resonant frequency of said resonant circuit changes to the radiation frequency of the one or more resonant RF fields comprised in said EM sequence block; and
- the resonant circuit has a frequency bandwidth smaller than the bandwidth range delimiting the values of the position-dependent Larmor frequency along the spatial extent of the subject or the sample along the at least one direction in said subject or said sample. In particular embodiments of the invention, the frequency bandwidth of the resonant circuit is at least two times smaller than said bandwidth range.

According to the above, by sweeping through frequencies comprised in the bandwidth range delimiting the above values of the position-dependent Larmor frequency, the NMR scanner is configured to successively excite the subject or the sample and acquire MR signal data from different portions of said subject or said sample. At the same time, the Q factor of the resonant circuit detecting the emitted MR signals is relatively large, as the frequency bandwidth of said resonant circuit is smaller than the bandwidth range delimiting the above values of the position-dependent Larmor frequency. Consequently, in these multifrequency embodiments, the SNR of the detected MR signals is relatively large. Moreover, the above Q factor (and therefore the above SNR), is particularly enhanced in multifrequency embodiments wherein the difference ($\Delta B_0$) between the maximum amplitude ($B_{0,max}$) and the minimum amplitude ($B_{0,min}$) of the non-uniform main magnetic field along the spatial extent of the subject or the sample along the at least one direction in said subject or said sample, is equal or larger than 0.5 times the maximum amplitude ($B_{0,max}$) of the non-uniform main magnetic field.

In addition, in particular multifrequency embodiments, wherein the NMR scanner is configured to detect the one or more emitted MR signals by means of a resonant circuit with a retunable resonant frequency, said NMR scanner is configured to steppedly execute, on the subject or the sample, the EM sequence blocks comprised in the EM sequence of step a), so that all radiation frequencies of the one or more resonant RF fields comprised in the EM sequence blocks, cover the whole the bandwidth range delimiting the values of the position-dependent Larmor frequency along the spatial extent of the subject or the sample along the at least one direction in said subject or said sample.

In further embodiments of the invention, wherein the EM sequence executed in step a) comprises a plurality of EM sequence blocks:

- the total number of EM sequence blocks comprised in the EM sequence is less than 100;
- in at least one pair of two consecutive EM sequence blocks comprised in the EM sequence, the repetition time between each of the first resonant RF fields of said at least one pair of two consecutive EM sequence blocks is less than 100 ms;
- the EM sequence blocks comprised in the EM sequence are different in strengths, directions, flip angles and/or repetition times; and/or
- the EM sequence blocks comprised in the EM sequence are randomly varied during step a). In particular embodiments of the invention, the EM sequence blocks comprised in the EM sequence are randomly varied by randomly varying the strengths, directions, flip angles and/or repetition times.

In particular embodiments of the invention, the total number of EM sequence blocks comprised in an EM sequence comprising a plurality of EM sequence blocks

is less than 90, less than 80, less than 70, less than 60, less than 50, less than 40, less than 30, less than 25, less than 20, less than 15, less than 10, less than 5, or less than 3; and/or
is equal to 90, 80, 70, 60, 50, 40, 30, 25, 20, 15, 10, 9, 8, 7, 6, 5, 4, 3, or 2.

In the embodiments of the invention wherein the total number of EM sequence blocks comprised in the EM sequence is less than 100, the scan times are reduced and the efficiency of the method for classifying MR signals is enhanced, with respect to MRI methods and known imageless methods for classifying MR signals. Said known MR methods typically comprise the execution of hundreds of different EM sequence blocks on a subject or the sample. In addition, said hundreds of different EM sequence blocks of known MR methods, also typically comprised a plurality of inhomogeneous magnetic field gradients, so that the acquired MR signal data sets are associated to k-space data sets of a k-space. These k-space data sets fill said k-space in different directions. In this sense, the execution of hundreds of different EM sequence blocks on a subject or a sample is particularly relevant in MRI methods to reconstruct a reliable image thereof.

On the other hand, in particular embodiments of the invention, the repetition time between each of the first resonant RF fields of at least one pair of two consecutive EM sequence blocks

is less than 90 ms, less than 80 ms, less than 70 ms, less than 60 ms, less than 50 ms, less than 40 ms, less than 30 ms, less than 25 ms, less than 20 ms, less than 15 ms, less than 10 ms, less than 5 ms, or less than 1 ms; and/or
is equal to 100 ms, 90 ms, 80 ms, 70 ms, 60 ms, 50 ms, 40 ms, 30 ms, 25 ms, 20 ms, 15 ms, 10 ms, 5 ms, or 1 ms.

In the embodiments of the invention where the repetition time between each of the first resonant RF fields of at least one pair of two consecutive EM sequence blocks is less than 100 ms, scan times are also reduced compared to MRI methods and known imageless methods. Indeed, in MRI methods repetition times of the order of 100 ms or larger are required, in order to prevent arftifacts in the reconstructed images. This is because said order of repetition times in MRI methods is sufficient to relax all the excited net magnetizations comprised in the excited portions of the subject/sample, as the excitation only takes place for each EM sequence block within a relatively narrow bandwidth range in said MRI methods (equal or smaller than 10 kHz).

As mentioned above, in particular embodiments of the invention, the EM sequence blocks comprised in the EM sequence are different in strengths, directions, flip angles and/or repetition times. Advantageously, the MR signal data sets acquired during the execution of the EM sequence look different from each other. These MR signal data sets that look different from each other may provide information about the different resonant species comprised in the subject or the sample (e.g., muscle, bone, blood, sclerosis lesions, etc). As used herein, the term "strength" refers to the maximum magnitude value of at least one inhomogeneous magnetic field gradient comprised in each of the EM sequence blocks. As used herein, the term "direction" refers to the direction with respect to a predefine reference frame of the subject or the sample of at least one inhomogeneous magnetic field gradient comprised in each of the EM sequence blocks. As used herein, the term "flip angle" refers to the angle to which a net magnetization is rotated or tipped via the execution of a resonant RF field comprised in an EM sequence block, relative to the direction of the main magnetic field applied in step a).

In particular embodiments of the invention, the one or more MR signal data sets and/or the reference data are non-image-reconstructing. As used herein, the term "non-image-reconstructing" means that from none group of the data comprised in the one or more MR signal data sets/reference data, is possible to reconstruct a reliable MRI image reproducing the internal spatial structure of a subject/sample. Hence, even after arranging said group of data into a 2D or 3D k-space and afterwards applying Fourier transformations, such reliable MRI image cannot be produced. In this regard, the one or more MR signal data sets and/or the reference data are non-image-reconstructing when at least one of the one or more MR signal data sets and/or the reference data only comprises 1D time-domain signal data.

In particular embodiments of the present invention, step b) is performed

- at full or quasi-full duty cycle; and/or
- during the execution of at least one resonant RF field comprised in the EM sequence executed in step a).

As used herein, the term "full duty cycle" refers to acquiring the at least part of each of the one or more emitted MR signals during a percentage of 100% of the time duration of the execution of the EM sequence executed in step a). On the other hand, as used herein, the term "quasi-full duty cycle" refers to acquiring the at least part of each of the one or more emitted MR signals during a percentage of less than 100% but at least 80% of the time duration of the execution of the EM sequence executed in step a).

When step b) is performed at full or quasi-full duty cycle, it is possible to measure the time evolution of the one or more MR signals during at least 80% of the time duration of the execution of the EM sequence executed in step a). In said embodiments of the invention, the NMR scanner operates at a duty cycle (between 100% and 80% duty cycle) considerably larger than in MRI methods and known imageless methods for classifying MR signals. Indeed, in said known MR methods the NMR scanners seldom operate at 50% duty cycle (even worse, in some cases they operate at duty cycles as low as 0.1%). Advantageously, when step b) is performed at full or quasi-full duty cycle, the amount of acquired MR signal data is enhanced, such that the SNR of the acquired MR signal data sets is increased with respect to above prior-art MR methods. Accordingly, the accuracy of the classification method according to said embodiments of the invention is enhanced, with respect to known imageless methods for classifying MR signals.

In further embodiments of the invention, the reference data comprise patterns and/or relationships comprising medical information, one or more threshold data values, and/or one or more critical data points (e.g., one or more relative and/or absolute maxima/minima), indicating the presence or absence, in the one or more reference subjects or samples, of one or more actual or potential future illnesses, symptoms, hurts, and/or physical features (e.g., amount of a specific fluid, tumor, and/or inflammation in the reference subjects or samples).

In particular embodiments of the invention, the reference data are correlated to one or more biomarkers indicating the presence or absence, in the one or more reference subjects or samples, of one or more actual or potential future illnesses, symptoms, hurts, and/or physical features. As used herein, the term "biomarker" refers to a measurable indicator of some biological state or condition of the reference subjects or samples.

In particular embodiments of the invention, the reference data comprise

- one or more MR signal data sets acquired with NMR scanner. In particular embodiment of the invention, said one or more MR signal data sets acquired with NMR scanner comprised in the reference data, are acquired following steps a) and b) of the present method, according to any of the realizations of said steps a) and b) disclosed in this document; and/or
- one or more simulated MR signal data sets.

In particular embodiments of the invention, step c), d) and/or e) is performed with an algorithm configured to recognised data patterns in MR signals data. In particular embodiments of the invention, said algorithm comprises the use of one or more predictive models, so that, based on data patterns recognition, the algorithm outputs one or more predictive outcomes from the one or more MR signal data sets inputted thereof. Furthermore, in particular non-limiting embodiments of the invention,

- the algorithm comprises the use of one or more predictive models including one or more regression models, time series models, clustering models, decision trees, ensemble models, artificial intelligence, AI, or machine learning, ML, models, and combinations of any of the foregoing, and/or
- the algorithm comprises the use of a pre-trained machine learning network. As used herein, a "pre-trained machine learning network" or "pre-trained ML network" is an AI or ML model based on a collection of connected units or nodes called artificial neurons, wherein said AI or ML model learns from training data to recognise patterns and/or relationships comprising relevant information.

In particular embodiments of the invention, the training data of the pre-trained ML network comprises data associated with the above one or more reference MR signals. Consequently, in these particular embodiments the reference data comprise patterns and/or relationships learnt by the pre-trained ML network from said data associated with the one or more reference MR signals. The learnt patterns and/or relationships comprises medical information associated with the above one or more reference subjects or samples.

Artificial neurons are commonly comprised in a plurality of layers. In particular embodiments of the invention, the pre-trained ML network comprises an input layer, one or more intermediate hidden layers, and/or one output layer. In particular embodiments of the invention, the one or more MR signal data sets are inputted to an input layer comprised in the pre-trained ML network. In particular embodiments of the invention, the one or more values of similarity determined in step d) comprise one or more predictive outcomes comprised in an output layer comprised in the pre-trained ML network. In particular embodiments of the invention, said one or more predictive outcomes weight the probability that the one or more emitted MR signals are associated with one or more of the above diagnostic or prognostic interest groups. In particular embodiments of the invention, the classification of the one or more MR signals emitted from the subject or the sample is derived from one or more predictive outcomes comprised in an output layer of the pre-trained ML network.

According to the present invention, a pre-trained ML network includes, but is not limited to, one or more multi-layer perceptron (MLPs), convolutional neural networks (CNNs), recurrent neural network (RNNs), and combinations of any of the foregoing.

In particular embodiments of the invention, the classification performed by the processing means in step e) comprises

- one or more binomial parameters that qualitatively indicate that the one or more MR signals emitted from the subject or the sample are associated to one or more of the above diagnostic or prognostic interest groups; and/or
- one or more scores that quantitatively indicate the degree of probability that the one or more MR signals emitted from the subject or the sample are associated to one or more of the above diagnostic or prognostic interest groups.

In particular embodiments of the invention, the EM sequence executed in step a) is selected according to a symptoms or condition database, wherein a specific EM sequence or combination of EM sequences are executed in step a) depending on a prescribed symptom or condition input information. In particular embodiments of the invention, said input information is inputted to the NMR scanner, or to the processing means or another means communicatively coupled with the NMR scanner.

In particular embodiments of the invention, the EM sequence executed in step a) is designed by an AI algorithm. This AI algorithm is configured to design said EM sequence according to the specific hardware resources of the NMR scanner used for realising the method, to the specific subject or specific sample arranged thereof, and/or to a specific a prescribed symptom or condition input information. Accordingly said designed EM sequence is optimal for the MR signals classification, given the above specific hardware resources of the NMR scanner, the specific subject or specific the sample, and/or specific a prescribed symptom or condition input information.

In addition, in particular embodiments of the invention:

- the EM sequences executed in step a) comprises one or more inhomogeneous magnetic field gradients, so that a position-dependent Larmor frequency is imposed along at least one direction in the subject or the sample. In particular embodiments of the invention, said one or more inhomogeneous magnetic field gradients comprise one or more inhomogeneous magnetic field gradient pulses;
- the EM sequence executed in step a) comprises one or more resonant RF fields comprising one or more RF pulses;
- the main magnetic field applied in step a) is a time-modulated main magnetic field.

In particular embodiments of the invention, the time-modulated main magnetic field is a cycling main magnetic field, such as, in a non-limiting example, a pre-polarization main magnetic field; and/or

- step b) is performed while the subject or the sample moves relative to the NMR scanner, or to a part thereof.

In a second aspect the present invention relates to a computer program comprising instructions which, when the computer program is executed by a processing means, cause the processing means to carry out a method according to the first aspect of the invention.

Finally, a third aspect the present invention relates to a system comprising an NMR scanner and a processing means communicatively coupled with the NMR scanner, wherein the NMR scanner and the processing means comprise hardware and/or software means adapted to perform a method according to the first aspect of the invention.

In particular embodiments of the invention, the processing means

- is communicatively coupled with the NMR scanner in wirelessly way and/or by means of one or more wires; and/or
- is integrated in the NMR scanner.

In particular embodiments of the invention, the NMR scanner comprises at least a main magnet, a RF transmitter, a RF receiver, and a data acquisition unit, wherein

- the main magnet is configured to apply a main magnetic field for polarising nuclear spins comprised in a subject or a sample, when said subject or said sample is arranged in the NMR scanner;
- the RF transmitter is configured to execute one or more resonant RF fields on said subject or said sample;
- the RF receiver is arranged to detect one or more MR signals emitted from said subject or said sample; and
- the data acquisition unit is configured to acquire at least part of each of the one or more MR signals detected by the RF receiver, by recording a set of one or more MR signal data for each of the one or more MR signals detected by the RF receiver.

In particular embodiments of the invention, the RF receiver comprises a resonant circuit. In these embodiments, the RF receiver detects MR signals through alternating currents induced by said MR signals in the resonant circuit. In particular embodiments of the invention, the RF receiver comprises a resonant circuit comprising at least one coil. In particular embodiments of the invention, the RF transmitter and the RF receiver are integrated in a same physical element (e.g., one or more coils).

Moreover, in particular embodiments of the invention the NMR scanner is adapted to realize the aforementioned steps a) and b) of a method according to any of the multifrequency embodiments described above. Thus, in particular embodiments of the invention, the NMR scanner comprises the above main magnet, RF transmitter and RF scanner, wherein

- the main field is a non-uniform main magnetic field that imposes a position-dependent Larmor frequency along the spatial extent of the subject or the sample along at least one direction in said subject or said sample, wherein the values of said position-dependent Larmor frequency along the spatial extent of the subject or the sample along the at least one direction in said subject or said sample are delimited by a bandwidth range ($\Delta w_L$) given by

$$(\gamma \, \Delta B_0)/(2\pi),$$

wherein $\Delta B_0$ is the difference ($\Delta B_0$) between the maximum amplitude ($B_{0,max}$) and the minimum amplitude ($B_{0,min}$) of the non-uniform main magnetic field along the spatial extent of the subject or the sample along the at least one direction in said subject or said sample, and $\gamma$ is the protonic gyromagnetic ratio;

- the RF transmitter is configured to execute, on the subject or the sample and during the application of the non-uniform main magnetic field, a plurality of resonant RF fields comprised in blocks, wherein each block comprises one or more RF fields with a radiation frequency, wherein said radiation frequency is different for different blocks and resonant with a single value of the position-dependent Larmor frequency along the spatial extent of the subject or the sample along the at least one direction in said subject or said sample;
- the RF receiver comprises a resonant circuit;
- said resonant circuit has a retunable resonant frequency, so that, for the detection of the emitted MR signal resulting from the execution of each block executed by the RF transmitter, the resonant circuit is configured to change the resonant frequency to the radiation frequency of the one or more resonant RF fields comprised in said block executed by the RF transmitter; and
- said resonant circuit has a frequency bandwidth smaller than the bandwidth range delimiting the values of the position-dependent Larmor frequency along the spatial extent of the subject or the sample along the at least one direction in said subject or said sample.

Since the above non-uniform main magnetic field already spatially encodes the subject or the sample, in the multifrequency embodiments it is not necessarily required a magnetic gradient system configured to execute one or more inhomogeneous magnetic field gradients for said spatial encoding of the subject or the sample. Hence, in particular multifrequency embodiments, the NMR scanner does not comprise said magnetic gradient system.

In particular multifrequency embodiments, the above RF transmitter is configured to steppedly execute the aforementioned blocks, so that all radiation frequencies of the one or more resonant RF fields comprised in said blocks cover the whole the bandwidth range delimiting the values of the above position-dependent Larmor frequency imposed by the main magnetic field. On the order hand, in particular multifrequency embodiments, the main magnet applying the non-uniform main magnetic field is a single-sided magnet. In further embodiments of the invention, the retunable resonant frequency of the resonant circuit is retunable by means one or more capacitors comprised in said resonant circuit, by changing the capacitance of each of the one or more capacitors. In non-limiting examples, the capacitance of a capacitor can be changed by switching said capacitor, and/or by adjusting voltages on, for example, varactor diodes.

Furthermore, in particular embodiments of the invention

- the NMR scanner comprises a magnetic gradient system configured to execute one or more inhomogeneous magnetic field gradients on a subject or a sample, when said subject or said sample is arranged in said NMR scanner, so that a position-dependent Larmor frequency is imposed along at least one direction in the subject or the sample. In particular embodiments of the invention, the magnetic gradient system comprises one or more coils;
- the NMR scanner is a resource-relaxed NMR scanner; and/or
- the NMR scanner is a non-image-reconstructing NMR scanner.

In the context of present invention, the term "resource-relaxed NMR scanner" refers to an NMR scanner with relaxed hardware resources, compared to common MRI scanners. Typically, common MRI scanners are configured to apply a highly homogeneous main magnetic fields and execute highly linear inhomogeneous magnetic field gradients on a subject or a sample, in order to reconstruct, from the subsequently acquired MR signal data sets, a high-quality image. Concretely, a resource-relaxed NMR scanner comprises:

- a main magnet configured to apply, on a subject or a sample, a main magnetic field with an amplitude ($Bo$) on the subject or the sample smaller than 1 T and/or with a difference ($\Delta B_0$) between the maximum amplitude ($B_{0,max}$) and the minimum amplitude ($B_{0,min}$) equal or larger than 0.5 times the maximum amplitude ($B_{0,max}$), when said subject or said sample is arranged in the NMR scanner; and/or
- a magnetic gradient system configured to execute, on a subject or a sample, at least one non-linear inhomogeneous magnetic field gradient, when said subject or said sample is arranged in the NMR scanner. As used herein, the term "non-linear inhomogeneous magnetic field gradient" means that, in the point of maximum deviation in the field of view of the subject or the sample, the magnetic field amplitude of said at least one inhomogeneous magnetic field gradient deviates a 20% or more, with respect to the maximum magnetic field amplitude of said at least one inhomogeneous magnetic field gradient. The term "field of view" or "FoV" refers to one or more portions of the subject or the sample from which the MR signal data are acquired.

In particular embodiments, the main magnet of the resource-relaxed NMR scanner is configured to apply a main magnetic field with an amplitude ($Bo$) on the subject or the sample smaller than 0.1 T.

The above undemanding hardware resources of resource-relaxed NMR scanners imply a considerable reduction of costs and an availability increase, compared to common MRI scanners. Advantageously, a system according to the invention comprising a resource-relaxed NMR scanner, may be sited in small clinics, outpatient departments, rural regions, low-income areas, etc.

On the other hand, as used herein, the term "non-image-reconstructing NMR scanner" refers to an NMR scanner that does not produce an image of a subject or a sample arranged thereof.

In particular embodiments of the invention, the processing means are communicatively coupled with the aforementioned data acquisition unit, so that the data acquisition unit provides to the processing means the one or more MR signal data sets acquired by the data acquisition unit.

In particular embodiments of the invention, the system comprises a display that outputs the classification of the one or more emitted MR signals performed by the processing means.

All the terms and embodiments described anywhere in this document are equally applicable to all aspects of the invention. It should be noted that, as used in the specification and in the appended claims, the singular forms "a", "an", and "the" include their plural referents unless the context clearly indicates otherwise.

**EXAMPLES**

The following invention is hereby described by way of the following examples, which are to be construed as merely illustrative and not limitative of the scope of the invention.

Example 1: Method flowchart

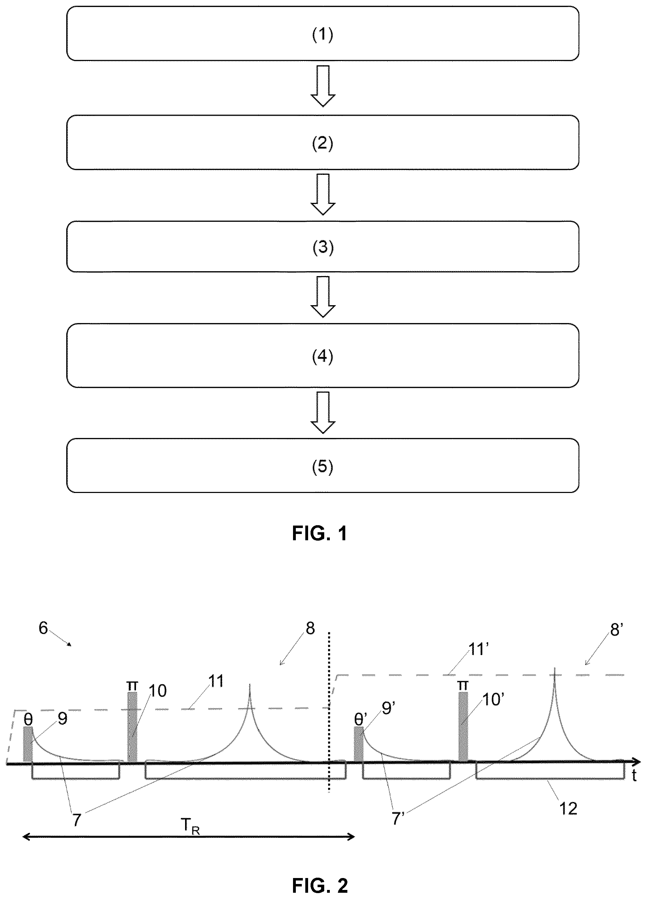

Figure 1 shows a flowchart comprising five stage-blocks (1)-(5) that schematically illustrate a particular embodiment of an imageless method for classifying MR signals according to present invention. In this example embodiment, a user that is a non-medical expert has a question of medical relevance that wants to address. In particular, the question of medical relevance is: "I have pain in the knee: is my anterior cruciate ligament torn?". In order to address said question, the user uses a system comprising a resource-relaxed NMR scanner and a processing means. The processing means is communicatively coupled with and integrated in the resource-relaxed NMR scanner.

In the above context, in the first stage-block (1) of Figure 1, the user inputs the question of medical relevance as a prescribed symptom input information to the system.

Then, in the second stage-block (2), the system, based on the prescribed symptom input information inputted in the first block (1), selects from a database an EM sequence to address the above question of medical relevance.

The method proceeds to the third stage-block (3), wherein the EM sequence selected in the second stage-block (2) is executed by the resource-relaxed NMR scanner on the user's pained knee arranged thereof. In addition, during the execution of the EM sequence, the resource-relaxed NMR scanner also applies a main magnetic field on the user's pained knee. Consequently, the user's pained knee emits MR signals. Furthermore, during the execution of the above EM sequence, a set of MR signal data is acquired with the resource-relaxed NMR scanner for at least part of each of said emitted MR signals. Contrary to prior-art MRI methods and imageless MR methods, said MR signal data sets acquired with the resource-relaxed NMR scanner comprise 1D time-domain signal data. Advantageously, although 1D time-domain signal data are not suitable for reconstructing an image of the user's knee, they comprise information associated with said user's knee that is indeed relevant for the classification of the above MR signals. Hence, the accuracy of the method according to present invention is enhanced with respect to prior-art imageless classification methods of MR signals.

In the fourth stage-block (4) of Figure 1, the resource-relaxed NMR scanner provides the MR signal data sets acquired in the third stage-blocks (3) to the processing means. The processing means is based on an algorithm configured to recognise data patterns in MR signal data information. In particular, said algorithm comprises the uses of a pre-trained ML network. The training data of said pre-trained ML network comprises data associated with one or more reference MR signals. More precisely, said training data are MR signal data sets previously acquired in response to a plurality of reference EM sequences executed on a plurality of reference knees. The training data are segmented into two groups:

- Group I: "knees with a torn anterior cruciate ligament";
- Group II: "knees without a torn anterior cruciate ligament".

Furthermore, contrary to prior-art imageless MR methods, the above training data comprise 1D time-domain signal data. In this context, in the fourth stage-block (4) of Figure 1, the processing means compares the one or more MR signal data sets acquired in the third stage-block (3) with the training data, so that the algorithm recognises patterns in said one or more MR signal data sets acquired in the third stage-block (3). Moreover, in the fourth stage-block (4) of Figure 1, the processing means also determines one or more values of similarity between the above two groups and the one or more MR signal data sets acquired in the third stage-block (3) of Figure 1.

Finally, in the fifth stage-block (5) of Figure 1, from the values of similarity determined in the fourth stage-block (4), the processing means classifies the MR signals emitted from the user's pained knee in either the Group I or Group II. Said classification comprises a binomial answer to the above question of medical relevance inputted in the first stage-block (1). The binomial answer is outputted by means of a display comprised in the system. In particular, the display outputs a "yes" answer when the MR signals are classified in the above Group I, or a "no" answer when the MR signals are classified in the above Group II.

In other non-limiting example embodiments of the invention, a question of medical relevance inputted to the system may be "is it the amount of fluid in the brain according to normal/expected levels in a subject with hydrocephalus", "which is the type of stroke in the brain of a subject", or "does the brain of a subject suffer from multiple sclerosis".

Example 2: execution of an EM sequences and acquisition of subsequent MR signal data

Figure 2 shows a schematic representation of an EM sequence (6) executed by an NMR scanner on a subject or a sample arranged thereof, in step a) of a non-limiting embodiment of the method according to present invention.

Figure 2 further shows two sets of MR signal data (7, 7') acquired, with the NMR scanner in step b) of the method, from two MR signals emitted from the subject or the sample. The MR signals result from the execution of above EM sequence (6), and from the application, with the NMR scanner during in step a), of a main magnetic field (not shown in Figure 2) that polarises nuclear spins comprised in the subject or the sample. Advantageously, the MR signal data sets (7, 7') comprise 1D time-domain signal data. In particular, in Figure 2 the MR signal data sets (7, 7') are voltage values recorded by a data acquisition unit comprised in the NMR scanner. Said voltage values are associated to the alternating currents induced by above MR signals in a resonant circuit comprised in the NMR scanner.

In Figure 2 a dotted vertical line separates two EM sequences blocks (8, 8') comprised in the EM sequence (6), and a horizontal arrow indicates the time (t) flow of the EM sequence (6).

The first EM sequence block (8) comprises two resonant RF pulses (9, 10) and an inhomogeneous magnetic field gradient pulse (11) imposing a position-dependent Larmor frequency along one direction in the subject or the sample. The first resonant RF pulse (9) of the first EM sequence block (8), sets an initial first flip angle ($\theta$) in the subject or the sample. That is to say, a net magnetization comprised in the subject or the sample, resulting from the application of the main magnetic field and aligned with the direction of said main magnetic field, tips by said fist flip angle ($\theta$) with respect to the direction of the main magnetic field. The second resonant RF pulse (10) is a $\pi$ resonant RF pulse executed later than the first RF pulse (9). This second resonant RF pulse (10) reverses by a $\pi$ the above tipped net magnetization. On the other hand, the inhomogeneous magnetic field gradient pulse (11) (represented with dashed lines in Figure 2) rises up to a certain strength and direction before the execution of the first resonant RF pulse (9). Hence, a first MR signal is emitted from the subject or the sample due to the execution of the first EM sequence block (8). Said first MR signal comprises a first RF radiation emitted from the subject or the sample after the execution of the first resonant RF pulse (9), and a second RF radiation emitted from the subject or the sample after the execution of the second resonant RF pulse (10). The second emitted RF radiation corresponds to a spin echo of the first MR signal. Indeed, the absolute maximum of the first MR signal data set (7) in Figure 2 is associated to said spin echo.

Similarly, the second EM sequence block (8') also comprises a first resonant RF pulse (9') setting an initial flip angle ($\theta'$), a second resonant $\pi$ RF pulse (10'), and an inhomogeneous magnetic field gradient pulse (11') (represented with dashed lines in Figure 2) imposing a position-dependent Larmor frequency along one direction in the subject or the sample. Analogously, a second MR signal is emitted from the subject or the sample due to the execution of the second EM sequence block (8'). The second emitted MR signal also comprises a first RF radiation emitted after the execution of the first resonant RF pulse (9'), and a second RF radiation emitted after the execution of the second resonant RF pulse (10'). Again, the second emitted RF radiation of the second emitted MR signal corresponds to a spin echo thereof.

However, the first resonant RF pulse (9') of the second EM sequence block (8') sets a second flip angle ($\theta'$) in the subject or the sample, which is different to the first flip angle ($\theta$) set by the first resonant RF pulse (9) of the first EM sequence (8). Moreover, the inhomogeneous magnetic field gradient field pulse (11') of the second EM sequence block (8') rises up to a strength and direction different to those of the inhomogeneous magnetic field gradient pulse (11) comprised in the first EM sequence block (8). Thus, the two EM sequences blocks (8, 8') are different in strengths, directions, and flip angles ($\theta$, $\theta'$). Consequently, the MR signal data sets (7, 7') associated to the emitted MR signals look different from each other.

Furthermore, in Figure 2, instead of ramping down the magnetic gradient field pulse (11) of the first EM sequence block (8) and letting sufficient time for the subject or the sample to de-excite, the second EM sequence block (8') is almost immediately executed on the sample or the subject. In particular, the repetition time ($T_R$) between the first resonant RF pulse (9) of the first EM sequence block (8) and the first resonant RF pulse (9') of the second EM sequence block (8') is equal to 1 ms. The time duration of said repetition time ($T_R$) is indicated with a double arrow in bottom side of Figure 2. Repetition times ($T_R$) of 1 ms are not implemented neither in MRI methods nor in known imageless MR methods. Accordingly, in the embodiment of Figure 2, scan times are advantageously reduced compared to said prior-art methods.

Coming back to the acquisition of data, in Figure 2 the acquisition time windows (12) when the acquisition of the MR signal data sets (7, 7') takes place are indicated with a plurality of rectangles, represented below the horizontal time-flow arrow. As it can be seen therein and contrary to common MRI methods and imageless MR methods, MR signal data are also acquired in acquisition time windows (12) that are not around spin echoes.

In addition, as it is also shown in Figure 2, the MR signal data sets (7, 7') are acquired almost throughout the whole-time duration of the EM sequence (6). In particular, step b) of the method is performed at quasi-full duty cycle, so that MR signal data sets (7, 7') are acquired during a percentage of less 100% but at least 80% of the execution time duration of the EM sequence (6). As mentioned above, in the best-case scenarios of MRI methods and known imageless MR methods, the MR signal data sets are acquired during a percentage of seldom more than 50% of the execution time duration of the EM sequence. Advantageously, in Figure 2 the amount of acquired MR signal data is enhanced so that the signal-to-noise ratio of the acquired MR signal data sets (7, 7') is increased, with respect to said prior-art methods. Accordingly, the accuracy of the classification method according to the embodiment of Figure 2, is enhanced compared to known imageless MR methods.

Example 3: execution of an EM sequences and acquisition of subsequent MR signal data (multifrequency embodiment)

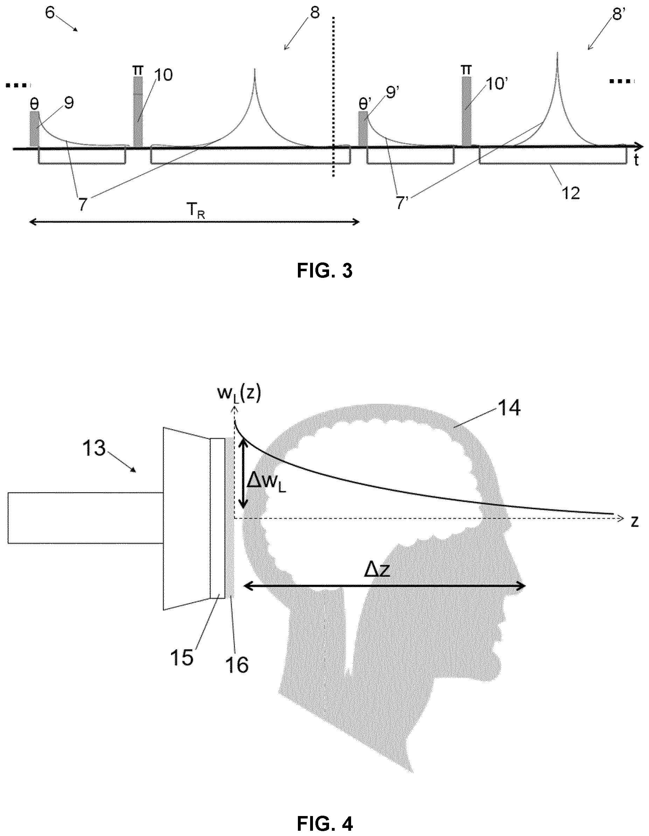

Similarly to Figure 2, Figure 3 shows a schematic representation of an EM sequence (6) and the MR signal data sets (7, 7') acquired according to an embedment of the invention. Indeed, in both Figures 2 and 3, elements that are the same or equivalent are indicated by the same reference numerals or Greek letters. However, in Figure 3 the EM sequence (6) corresponds to a non-limiting multifrequency embodiment, so that the NMR scanner applies a non-uniform main magnetic field (not shown in Figure 3) on the subject or the sample. Consequently, a position-dependent Larmor frequency is imposed along the spatial extent of the subject or the sample along one direction in said subject or said sample. Without loss of generality, herein said one direction is set to the z direction.

The values of the above position-dependent Larmor frequency are delimited by a bandwidth range ($\Delta w_L$) given by

$$(\gamma \, \Delta B_0)/(2\pi),$$

wherein "$\Delta B_0$" is the difference between the maximum amplitude ($B_{0,max}$) and the minimum amplitude ($B_{0,min}$) of the non-uniform main magnetic field along the spatial extent of the subject or the sample along the z direction, and "$\gamma$" is the protonic gyromagnetic ratio ($\gamma = 2.68 \times 10^8$ rad T$^{-1}$ s$^{-1}$).

The EM sequence (6) of the embodiment of Figure 3 comprises N sequence blocks (8, 8'). Figure 3 shows the n$^{th}$ EM block (8) and the (n+1)$^{th}$ EM block (8') comprised in said EM sequence (6). The dots represented in both left- and right-handed sides of Figure 3 indicate that more EM sequence blocks are comprised in the EM sequence (6) aside from those shown therein. Similarly to the EM sequence (6) of Figure 2, both n$^{th}$ and (n+1)$^{th}$ EM bocks (8, 8) comprise a first resonant RF pulses (9, 9') setting an initial flip angle ($\theta$, $\theta'$) and a second resonant $\pi$ RF pulse (10, 10'). However, none of the EM sequence blocks (8, 8') of the entire EM sequence (6) comprise magnetic gradient fields, as the spatial encoding of the subject or the sample is already performed by the non-uniform main magnetic field.

In the embodiment of Figure 3, the NMR scanner also comprises a resonant circuit. In particular, the resonant circuit has a retunable resonant frequency. Hence, to detect the MR signal resulting from the execution of each EM sequence block (8, 8'), the resonant frequency of said resonant circuit changes to the radiation frequency of the one or more resonant RF pulses (9, 9') comprised in said EM sequence block (8, 8'). In addition, the resonant circuit has a frequency bandwidth ($\Delta w_c$) smaller than the delimiting bandwidth range ($\Delta w_L$) imposed on the subject or the sample by the non-uniform main magnetic field. In this particular embodiment, the frequency bandwidth ($\Delta w_c$) of the resonant circuit is given by

$$\Delta w_c = \Delta w_L \, /N,$$

wherein "N" is the total number of EM sequence blocks (8, 8') comprised in the EM sequence (6).

In the above context, the spatial extent encoded by the non-uniform magnetic field along the z direction in the subject or the sample can be seen as split into N different portions. The EM sequence (6) steppedly sweeps the resonant frequency of the resonant circuit across the whole delimiting bandwidth range ($\Delta w_L$) imposed by the non-uniform main magnetic field. In this respect, between two consecutive EM sequence blocks (8, 8'), the difference between the radiation frequency of the resonant pulses RF pulses (9, 9', 10, 10') of the later EM sequence block (8, 8'), and the radiation frequency of the resonant pulses RF pulses (9, 9', 10, 10') of the earlier EM sequence block (8, 8'), is equal to frequency bandwidth ($\Delta w_c$) of the resonant circuit. In addition, the associated resonant frequency of the resonant circuit changes by an amount equal to the frequency bandwidth ($\Delta w_c$) of the resonant circuit.

According to the above, for instance, in the n$^{th}$ EM sequence block (8) of Figure 3, the resonant frequency of both the first and second resonant RF pulses (9, 10) is given by

$$w_{RF}^{(n)} = w_{L,max} - n \, \Delta w_c,$$

and the associated resonant frequency of the resonant circuit in the detection of the subsequent n$^{th}$ emitted MR signal is given by,

$$w_c^{(n)} = w_{L,max} - n \, \Delta w_c.$$

Herein, "$w_{L,max}$" is the maximum value of the position-dependent Larmor frequency along the spatial extent of the subject or the sample along the z direction. On the other hand, in the (n+1)$^{th}$ EM sequence block (8') both the resonant frequency of the first and second resonant RF pulses (9', 10'), and the respective associated resonant frequency of the resonant circuit, increase by an amount equal to the frequency bandwidth ($\Delta w_c$) of the resonant circuit.

Hence, in the embodiment of Figure 3, the N EM sequence blocks (8, 8') comprised in the EM sequence (6) cover the whole delimiting bandwidth range ($\Delta w_L$) imposed by the non-uniform main magnetic field. In this sense, the NMR scanner applies the non-uniform main magnetic field, executes the EM sequence (6), and acquires the MR signal data sets (7, 7'), so that it successively excites different portions of the subject or the sample and acquires the MR signals from said different portions.

Example 4: NMR scanner comprising a single-side magnet

Figure 4 shows a schematic representation of an NMR scanner (13) in a non-limiting multifrequency embodiment according to the invention. Said NMR scanner (13) is a handheld NMR scanner configured to apply a main magnetic field and execute EM sequences on a sample (14), and to acquire the subsequent MR signals resulting thereof. To this end, NMR scanner (13) comprises a main magnet (15), a RF system (16), and a data acquisition unit (not shown in Figure 4).

The main magnet (15) is a single-sided magnet configured to apply a non-uniform main magnetic field, so that a position-dependent Larmor frequency is imposed along the spatial extent of the sample (14) in the z direction. The values of said position-dependent Larmor frequency are delimited by a bandwidth range ($\Delta w_L$) given by

$$(\gamma\,\Delta B_0)/(2\pi),$$

wherein $\Delta B_0$ is the difference ($\Delta B_0$) between the maximum amplitude ($B_{0,max}$) and the minimum amplitude ($B_{0,min}$) of the non-uniform main magnetic field along the spatial extent of the sample (14) in the z direction.

Figure 4 shows a graphic superimposed over the subject (14). Said graphic indicates how the position-dependent Larmor frequency changes along the z direction. Therein, both the spatial extent ($\Delta z$) of the sample (14) in the z direction and the delimiting bandwidth range ($\Delta w_L$) along said spatial extent ($\Delta z$), are highlighted by two double arrows.

Concerning the RF system (16) of Figure 4, it comprises a RF system and a RF receiver. In particular, the RF transmitter and the RF receiver are integrated in a same resonant circuit comprising a coil. The resonant circuit has a frequency bandwidth smaller than the above delimiting bandwidth range ($\Delta w_L$). Advantageously, the Q factor of said resonant circuit is relatively large, given the above delimiting bandwidth range ($\Delta w_L$).

The RF system (16) is configured to work in a transmitter mode and in a receiver mode. Hence, when working in the transmitter mode, the RF system (16) is a RF transmitter configured to execute, on the sample (14) and during the application of the non-uniform main magnetic field, a plurality of resonant RF fields comprised in blocks. Each block comprises one or more RF fields with a radiation frequency, wherein said radiation frequency is different for different blocks and resonant with a single value of the position-dependent Larmor frequency along the z direction of the sample (14).

On the other hand, when working in the receiver mode, the RF system (16) is a RF receiver configured to retune the resonant frequency of the resonant circuit comprised thereof. Accordingly, to detect the MR signal resulting from each block executed by the RF transmitter, the resonant frequency of said resonant circuit changes to the radiation frequency of the one or more resonant RF fields comprised in said block.

Finally, the data acquisition unit of the NMR scanner (13), is configured to acquire at least part of each of the one or more MR signals detected by the RF receiver, by recording a set of MR signal data for each of said one or more detected MR signals. According to the present invention, the one or more MR signal data sets advantageously comprise 1D time-domain signal data, contrary to known imageless MR methods. In addition, the data acquisition unit is communicatively coupled to a processing means, so that the data acquisition unit provides to said processing means the one or more MR signal data sets. The processing means processes said one or more MR signal data sets, to classify the above one or more MR signals in one or more diagnostic or prognostic interest groups.

**Claims**

1. A method of classification of magnetic resonance, MR, signals indicative of one or more portions of a subject or a sample of diagnostic or prognostic interest, comprising the realization, by means of an NMR scanner (13) and a processing means, of the following steps:

   a) applying a main magnetic field and executing a sequence of electromagnetic (EM) fields (6), on the subject or the sample (14) arranged in the NMR scanner;
   b) acquiring with the NMR scanner (13), during the application of the main magnetic field and the execution of the EM sequence (6) in step a), at least part of each of the one or more MR signals emitted from the subject or the sample (14) that result from the application of the main magnetic field and the execution of the EM sequence (6), so that a set of MR signal data (7, 7') is acquired for said each of the one or more emitted MR signals;

c) comparing, with the processing means, the one or more MR signal data sets (7, 7') of step b) with reference data, wherein said reference data are associated with one or more reference MR signals associated with one or more reference EM sequences for one or more reference subjects or samples, and wherein said reference data is segmented into a plurality of diagnostic or prognostic interest groups;

d) determining, with the processing means, one or more values of similarity between the diagnostic or prognostic interest groups and the one or more MR signal data sets;

e) classifying, with the processing means, the one or more MR signals emitted from the subject or the sample in one or more diagnostic or prognostic interest groups of the reference data from the values of similarity determined in step d);

the method **being characterising in that**

- the one or more MR signal data sets (7, 7') comprise one-dimensional time-domain signal data; and
- the reference data associated with the one or more reference MR signals comprise one-dimensional time-domain signal data.

**2.** .- The method according to claim 1, wherein the EM sequence (6) executed in step a) comprises a plurality of EM sequence blocks (8, 8'), wherein each EM sequence block (8, 8') comprises one or more resonant RF fields (9, 9', 10, 10'), so that the subject or the sample (14) is repeatedly re-excited during step a).

**3.** .- The method according to claim 2, wherein

- the main magnetic field applied in step a) is a non-uniform main magnetic field, so that a position-dependent Larmor frequency is imposed along the spatial extent ($\Delta z$) of the subject or the sample (14) along at least one direction in said subject or said sample (14), wherein the values of said position-dependent Larmor frequency along the spatial extent ($\Delta z$) of the subject or the sample (14) along said at least one direction are delimited by a bandwidth range ($\Delta w_L$) given by

$$(\gamma\,\Delta B_0)/(2\pi),$$

wherein $\Delta B_0$ is the difference ($\Delta B_0$) between the maximum amplitude ($B_{0,max}$) and the minimum amplitude ($B_{0,min}$) of the non-uniform main magnetic field along the spatial extent ($\Delta z$) of the subject or the sample (14) along the at least one direction in said subject or said sample (14), and $\gamma$ is the protonic gyromagnetic ratio;

- the one or more RF fields (9, 9', 10, 10') of each of the EM sequence blocks (8, 8') has a radiation frequency, wherein said radiation frequency is different for different EM sequence blocks (8, 8') and resonant with a single value of the position-dependent Larmor frequency along the spatial extent ($\Delta z$) of the subject or the sample (14) along the at least one direction in said subject or sample (14).

**4.** .- The method according to claims 3, wherein

- step b) comprises the record of the at least part of each of the one or more emitted MR signals detected by the NMR scanner (13) by means of resonant circuit;
- the resonant circuit has a retunable resonant frequency, so that, for the detection of the emitted MR signal resulting from the execution of each EM sequence block (8, 8'), the resonant frequency of said resonant circuit changes to the radiation frequency of the one or more resonant RF fields (9, 9', 10, 10') comprised in said EM sequence block (8, 8'); and
- the resonant circuit has a frequency bandwidth smaller than the bandwidth range ($\Delta w_L$) delimiting the values of the position-dependent Larmor frequency along the spatial extent ($\Delta z$) of the subject or the sample (14) along the at least one direction in said subject or said sample (14).

**5.** .- The method according to any one of claims 2 to 4, wherein:

- the total number of EM sequence blocks (8, 8') comprised in the EM sequence (6) is less than 100;
- in at least one pair of two consecutive EM sequence blocks (8, 8') comprised in the EM sequence (6), the repetition time ($T_R$) between each of the first resonant RF fields (9, 9') of said at least one pair of two consecutive EM sequence blocks (8', 8') is less than 100 ms;

- the EM sequence blocks (8, 8') comprised in the EM sequence (6) are different in strengths, directions, flip angles ($\theta$, $\theta$') and/or repetition times ($T_R$); and/or
- the EM sequence blocks (8, 8') comprised in the EM sequence (6) are randomly varied during step a).

6. .- The method according to any one of claims 1 to 5, wherein the one or more MR signal data sets are non-image-reconstructing.

7. .- The method according to any one of claims 1 to 6, wherein step b) is performed

- at full or quasi-full duty cycle; and/or
- during the execution of at least one resonant RF field (9, 9') comprised in the EM sequence (6) executed in step a).

8. .- The method according to any one of claims 1 to 7, wherein step c), d) and/or e) is performed with an algorithm configured to recognised data patterns in MR signals data.

9. .- The method according to claim 8, wherein

- the algorithm comprises the use of one or more predictive models including one or more regression models, time series models, clustering models, decision trees, ensemble models, AI or ML models, and combinations of any of the foregoing, and/or
- the algorithm comprises the use of a pre-trained machine learning network.

10. .- The method according to any one of claims 1 to 9, wherein the EM sequence (6) executed in step a) is selected according to a symptoms or condition database, wherein a specific EM sequence (6) or combination of EM sequences (6) are executed in step a) depending on a prescribed symptom or condition input information.

11. .- A computer program comprising instructions which, when the computer program is executed by a processing means, cause the processing means to carry out a method according to any one of claims 1 to 10.

12. .- A system comprising an NMR scanner (13) and a processing means communicatively coupled with the NMR scanner (13), wherein the NMR scanner (13) and the processing means comprise hardware and/or software means adapted to perform a method according to any one of claims 1 to 10.

13. .- The system according to claim 12, wherein the NMR scanner (13) comprises at least a main magnet (15), a RF transmitter (16), a RF receiver (16), and a data acquisition unit, wherein

- the main magnet (15) is configured to apply a main magnetic field for polarising nuclear spins comprised in a subject or a sample (14), when said subject or said sample (14) is arranged in the NMR scanner (13);
- the RF transmitter (16) is configured to execute one or more resonant RF fields (9, 9', 10, 10') on said subject or said sample (14);
- the RF receiver (16) is arranged to detect one or more MR signals emitted from said subject or said sample (14); and
- the data acquisition unit is configured to acquire at least part of each of the one or more MR signals detected by the RF receiver, by recording a set of one or more MR signal data (7, 7') for each of the one or more MR signals detected by the RF receiver.

14. .- The system according to claim 13, wherein

- the main field is a non-uniform main magnetic field that imposes a position-dependent Larmor frequency along the spatial extent ($\Delta z$) of the subject or the sample (14) along at least one direction in said subject or said sample (14), wherein the values of said position-dependent Larmor frequency along the spatial extent ($\Delta z$) of the subject or the sample (14) along the at least one direction in said subject or said sample (14) are delimited by a bandwidth range ($\Delta w_L$) given by

$$(\gamma \, \Delta B_0)/(2\pi),$$

wherein $\Delta B_0$ is the difference between the maximum amplitude ($B_{0,max}$) and the minimum amplitude ($B_{0,min}$) of the non-uniform main magnetic field along the spatial extent ($\Delta z$) of the subject or the sample (14) along the at least one direction in said subject or said sample (14), and $\gamma$ is the protonic gyromagnetic ratio;

- the RF transmitter (16) is configured to execute, on the subject or the sample (14) and during the application of the non-uniform main magnetic field, a plurality of resonant RF fields (9, 9', 10, 10') comprised in blocks, wherein each block comprises one or more RF fields (9, 9', 10, 10') with a radiation frequency, wherein said radiation frequency is different for different blocks and resonant with a single value of the position-dependent Larmor frequency along the spatial extent ($\Delta z$) of the subject or the sample (14) along the at least one direction in said subject or said sample (14);

- the RF receiver (16) comprises a resonant circuit;

- said resonant circuit has a retunable resonant frequency, so that, for the detection of the emitted MR signal resulting from the execution of each block executed by the RF transmitter (16), the resonant circuit is configured to change the resonant frequency to the radiation frequency of the one or more resonant RF fields (9, 9', 10, 10') comprised in said block executed by the RF transmitter (16); and

- said resonant circuit has a frequency bandwidth smaller than the bandwidth range ($\Delta w_L$) delimiting the values of the position-dependent Larmor frequency along the spatial extent ($\Delta z$) of the subject or the sample (14) along the at least one direction in said subject or said sample (14).

15. .- The system according to any one of claims 12 to 14, wherein

- the NMR scanner (13) comprises a magnetic gradient system configured to execute one or more inhomogeneous magnetic field gradients on a subject or a sample (14), when said subject or said sample (14) is arranged in said NMR scanner (13), so that a position-dependent Larmor frequency is imposed along at least one direction in the subject or the sample (14);

- the NMR scanner (13) is a resource-relaxed NMR scanner; and/or

- the NMR scanner (13) is a non-image-reconstructing NMR scanner.

**FIG. 1**

**FIG. 2**

**FIG. 3**

**FIG. 4**

Europäisches
Patentamt

European
Patent Office

Office européen
des brevets

## EUROPEAN SEARCH REPORT

**Application Number**

EP 23 38 3313

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | RAGHAV SINGHAL ET AL: "On the Feasibility of Machine Learning Augmented Magnetic Resonance for Point-of-Care Identification of Disease", ARXIV.ORG, CORNELL UNIVERSITY LIBRARY, 201 OLIN LIBRARY CORNELL UNIVERSITY ITHACA, NY 14853, 2 February 2023 (2023-02-02), XP091427285, * the whole document * | 1-15 | INV. G01R33/56 A61B5/055 G06F18/24 |
| A | CN 114 494 484 A (SHENYANG DONGXIA INTELLIGENT MEDICAL SCIENCE AND TECH RESEARCH INSTITU) 13 May 2022 (2022-05-13) * the whole document * | 1-15 | |

**TECHNICAL FIELDS SEARCHED (IPC)**

G01F
G06F
G01R
G06V

The present search report has been drawn up for all claims

| Place of search | Date of completion of the search | Examiner |
|---|---|---|
| Munich | 4 June 2024 | Vanhaecke, Nicolas |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 38 3313

This annex lists the patent family members relating to the patent documents cited in  the above-mentioned European search report.
The members are as contained in the European Patent Office EDP file on
The European Patent Office is in no way liable for these particulars which are merely given for the purpose of information.

04-06-2024

| Patent document cited in search report | Publication date | Patent family member(s) | Publication date |
|---|---|---|---|
| CN 114494484 A | 13-05-2022 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019051411 A1

**Non-patent literature cited in the description**

- **SINGHAL et al.** On the Feasibility of Machine Learning Augmented Magnetic Resonance for Point-of-Care Identification of Disease. *arXiv:2301.11962*, 2023